# EUROPEAN PATENT APPLICATION

(11) **EP 4 311 551 A1**
(43) Date of publication of application: **31.01.2024**
(21) Application number: 23186654.2
(22) Date of filing: 20.07.2023
(51) Int. Cl.: A61K 31/46, A61P 27/10

(54) **LOW-CONCENTRATION ATROPINE FOR MYOPIA PREVENTION (LAMP-2) STUDY**

(30) Priority: 26.07.2022 US 202217814911
(71) Applicant: The Chinese University of Hong Kong, Shatin, N.T. Hong Kong (CN)
(72) Inventor: Yam, Cheuk Sing, Hong Kong (CN); Pang, Chi Pui, Hong Kong (CN); Chen, Lijia, Hong Kong (CN); Zhang, Xiujuan, Hong Kong (CN); Tham, Chee Yung Clement, Hong Kong (CN)
(74) Representative: HGF

(57) **Abstract**

The subject invention pertains to compositions comprising atropine at a concentration of greater than 0.025% to about 0.05% and methods of administering said compositions to a subject to inhibit or delay the onset of myopia in pre-myopic patients.

## Description

### BACKGROUND OF THE INVENTION

Myopia is a worldwide public health issue, and is highly prevalent in Hong Kong [1-3]. It is predicted that around half and one-tenth of the world's population will become myopic and highly myopic respectively by year 2025 [4, 5]. Highly myopic individual has excessive eyeball growth and therefore a higher risk of sight-threatening complications, including glaucoma, cataract, retinal detachment, and other stretch-induced degenerative changes of retinal pigment epithelium, choroid and sclera [6]. In China, about 36.5% fourth graders and 65.3% eighth graders have poor eyesight [7]. Prevention of childhood myopia progression is exceedingly important. In view of that, President Xi Jinping has stated the need to control myopia in China for a better children's eye health and bright future [8]. A national scheme, jointly issued by Ministry of Education, the National Health Commission and six other Departments of the state, has been announced to reduce the myopia rate among 6 years old to 3%, among primary school pupils to below 38%, junior high school pupils to below 60% and senior high school students to 70%by year 2030 [9].

Despite decades of scientific research in the causes of myopia, its exact etiology remains unclear. Environmental pressures, such as the rigorous education system in highly urbanized cities like Hong Kong that demand heavy near work from intensive reading papers or electronic screens, are attributed to the high prevalence of myopia. Electronic device usage is inevitable in children in Hong Kong nowadays. Furthermore, the prevalence of myopia in Chinese is the highest among all populations in the world. [4,5] It is notable that children start to develop myopia as early as age of four, with fast progression from age eight to twelve, and then a slower progression until around age eighteen. Myopia, once developed and progressed, is irreversible. Therefore, timely measures to prevent myopia onset and its progression since childhood is exceedingly crucial [7].

Different interventions have been attempted to reduce myopic progression, such as increasing outdoor time [10-12]. Other methods include optical methods, e.g., bifocal/progressive spectacles [13-16], orthokeratology [17, 18], defocus spectacles and contact lens [19]. Pharmacological methods, mainly atropine eye drops, have been shown to have some effects [20-24]. A Cochrane database systemic review concluded that anti-muscarinic agents including atropine eye drop were the most effective anti-myopia treatment [25]. A meta-analysis of 16 different interventions also showed that atropine eye drops conferred the best efficacy among all myopia prevention methods [26]. The American Academy of Ophthalmology also recommended its use [27].

Atropine is a nonselective muscarinic receptor antagonist with many postulated mechanisms for its anti-myopia actions, including its biochemical effects on the retina and sclera for eyeball remodeling [28, 29]. Atropine in the Treatment of Myopia (ATOM 1) study at a daily regimen of 1% atropine eye drops reduced myopia progression by 77% when compared with placebo eye drops treatment over a 2-year period [21]. However, side effects included cycloplegia and pupil dilatation resulting in blurred near vision and photophobia. The subsequent ATOM 2 study from the same study group showed that lower concentrations of topical atropine (0.5%, 0.1% and 0.01%) one drop nightly over two years were effective in reducing annual myopia progression to -0.3+/-0.6D, -0.38+/-0.6D, and -0.49+/-0.63D, respectively [22]. Because of low side effects and less rebound after stopping the treatment, 0.01% atropine was recommended as the optimal concentration [20]. The use of a low-concentration atropine is further supported by other groups from the United States [30] and Taiwan [31-34].

Unfortunately, after the onset of myopia in children, its progression is very difficult to control. Currently, there is no effective intervention for inhibiting or delaying the onset of myopia. In a retrospective study of 24 children, a composition comprising 0.025% atropine was administered to myopic children nightly versus 26 children in the control group without any intervention, in which there was a significantly lower proportion of onset of myopia along the one-year follow-up period in the treatment group versus the control group (21% vs 54%) [35]. This study was however limited by its retrospective nature and small sample size [35].

Therefore, there remains a need for a safe and effective treatment to inhibit or delay the onset of myopia, particularly childhood myopia.

### BRIEF SUMMARY OF THE INVENTION

The present invention relates to a nonselective muscarinic receptor antagonist, atropine, and compositions comprising atropine. In certain embodiments, the concentration of atropine in the composition can be about 0.001% to about 0.5%, preferably about 0.05% to about 0.01%. The present invention further pertains to novel methods of inhibiting or delaying the onset of myopia, particularly in children. In certain embodiments, the children are less than about 12, about 11, about 10, about 9, about 8, about 7, about 6, about 5, or about 4 years old. In certain embodiments, administration of atropine compositions can occur for at least about six months, about one year, about two years, about three years, about four years, about five years, about six years, about seven years, or about eight years.

In certain embodiments, the Spherical Equivalent (SE) of the eye is about +1.00 D to greater than -0.5 D before administration of the composition. In some embodiments, the SE is measured by cycloplegic autorefraction. In certain embodiments, the subject has no astigmatism or has astigmatism of less than about 1.00 D. In certain embodiments, the subject has no anisometropia or has anisometropia of less than about 2.00 D. In certain embodiments, the subject has at least one myopic parent. The myopic subject can have an SE of less than or equal to -3.00 D.

In certain embodiments, the onset of myopia is inhibited or delayed. In certain embodiments, the onset of myopia is delayed for at least about 1 month, about 2 months, about 3 months, about 4 months, about 5 months, about 6 months, about 12 months, about 18 months, about two years, about three years, about 4 years, about five years, about six years, about 7 years, or about eight years.

### BRIEF DESCRIPTION OF THE DRAWINGS

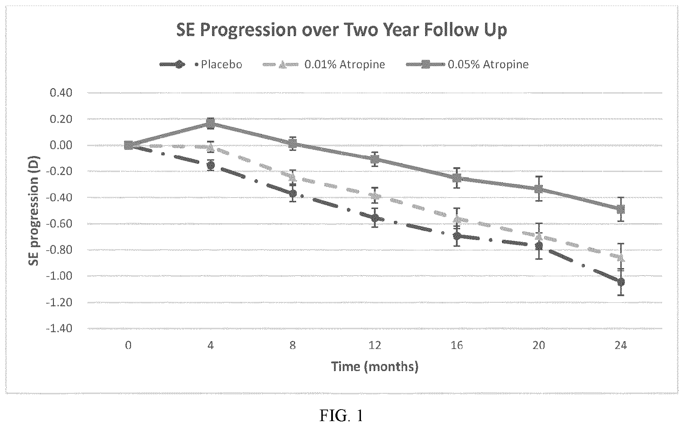

**FIG. 1** Change in spherical equivalent (SE) in treatment groups across time.

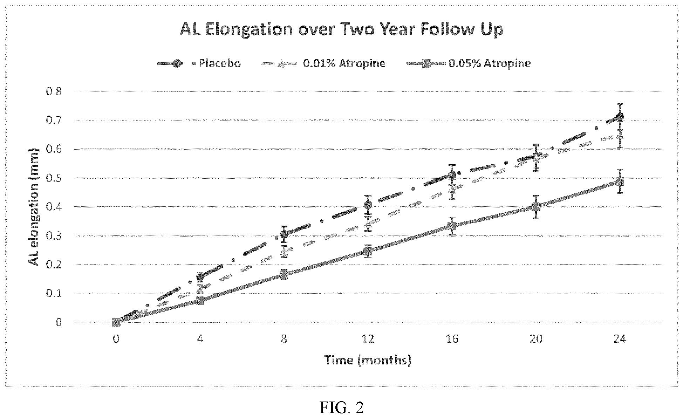

**FIG. 2** Change in axial length (AL) in treatment groups across time.

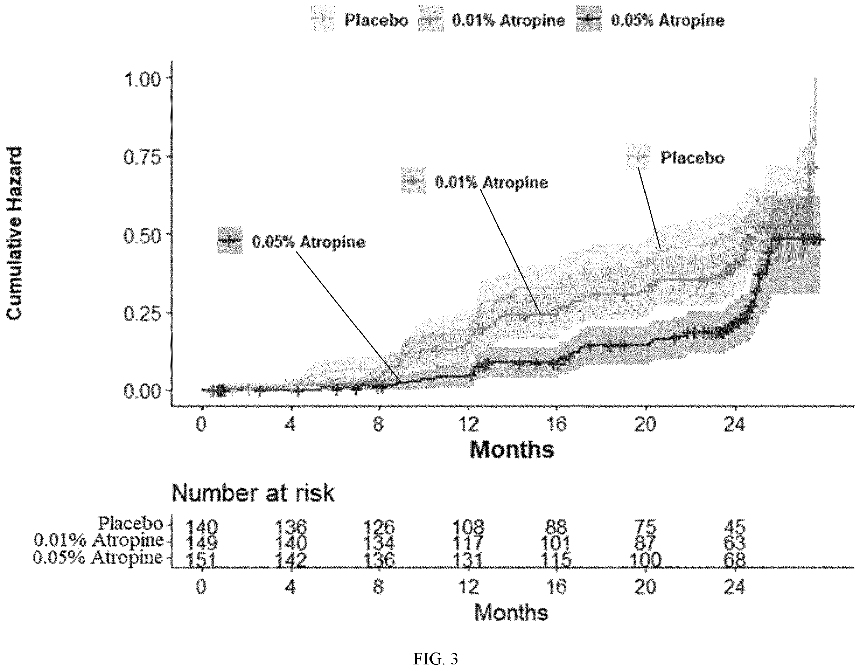

**FIG. 3** Cumulative hazards estimated for 0.05% and 0.01% atropine groups and placebo group.

### DETAILED DISCLOSURE OF THE INVENTION

### Selected Definitions

As used herein, the singular forms "a," "an," and "the" are intended to include the plural forms as well, unless the context clearly indicates otherwise. Further, to the extent that the terms "including," "includes," "having," "has," "with," or variants thereof are used in either the detailed description and/or the claims, such terms are intended to be inclusive in a manner similar to the term "comprising." The transitional terms/phrases (and any grammatical variations thereof) "comprising," "comprises," "comprise," include the phrases "consisting essentially of," "consists essentially of," "consisting," and "consists."

The phrases "consisting essentially of' or "consists essentially of' indicate that the claim encompasses embodiments containing the specified materials or steps and those that do not materially affect the basic and novel characteristic(s) of the claim.

The term "about" means within an acceptable error range for the particular value as determined by one of ordinary skill in the art, which will depend in part on how the value is measured or determined, *i.e.,* the limitations of the measurement system. Where particular values are described in the application and claims, unless otherwise stated the term "about" meaning within an acceptable error range for the particular value should be assumed.

In the present disclosure, ranges are stated in shorthand, to avoid having to set out at length and describe each and every value within the range. Any appropriate value within the range can be selected, where appropriate, as the upper value, lower value, or the terminus of the range. For example, a range of 1-10 represents the terminal values of 1 and 10, as well as the intermediate values of 2, 3, 4, 5, 6, 7, 8, 9, and all intermediate ranges encompassed within 1-10, such as 2-5, 2-8, and 7-10. Also, when ranges are used herein, combinations and sub-combinations of ranges (e.g., subranges within the disclosed range) and specific embodiments therein are intended to be explicitly included.

In certain embodiments of the invention a subject is a mammal. Non-limiting examples of a mammal treatable according to the methods of the current invention include mouse, rat, dog, guinea pig, cow, horse, cat, rabbit, pig, monkey, ape, chimpanzee, and human. Additional examples of mammals treatable with the methods of the current invention are well known to a person of ordinary skill in the art and such embodiments are within the purview of the current invention.

For the purposes of this invention the terms "treatment, treating, treat" or equivalents of these terms refer to healing, alleviating, relieving, altering, remedying, ameliorating, improving, or affecting the condition or the symptoms of a subject suffering with a disease or condition, for example, a myopia. The subject to be treated can be suffering from or at risk of developing the disorder or condition, for example, myopia. When provided therapeutically, the compound can be provided before the onset of a symptom. The therapeutic administration of the substance serves to attenuate any actual symptom.

For the purposes of this invention, the terms "preventing, preventive, prophylactic" or equivalents of these terms are indicate that the compounds of the subject invention are provided in advance of any disease symptoms and are a separate aspect of the invention (i.e., an aspect of the invention that is distinct from aspects related to the terms "treatment, treating, treat" or equivalents of these terms which refer to healing, alleviating, relieving, altering, remedying, ameliorating, improving, or affecting the condition or the symptoms of a subject suffering from myopia). The prophylactic administration of the compounds of the subject invention serves to prevent, reduce the likelihood, or attenuate one or more subsequent symptoms or condition.

By "therapeutically effective dose," "therapeutically effective amount", or "effective amount" is intended to be an amount of a compounds of the subject invention disclosed herein that, when administered to a subject, decreases the number or severity of symptoms or inhibits or eliminates the progression or initiation of myopia or reduces any increase in symptoms, or improve the clinical course of the disease as compared to untreated subjects. "Positive therapeutic response" refers to, for example, improving the condition of at least one of the symptoms of myopia.

An effective amount of the therapeutic agent is determined based on the intended goal. The term "unit dose" refers to a physically discrete unit suitable for use in a subject, each unit containing a predetermined quantity of the therapeutic composition calculated to produce the desired response in association with its administration, i.e., the appropriate route and treatment regimen. The quantity to be administered, both according to number of treatments and unit dose, depends on the subject to be treated, the state of the subject and the protection desired. Precise amounts of the therapeutic composition also depend on the judgment of the practitioner and are peculiar to each individual. Generally, the dosage of the compounds of the subject invention will vary depending upon such factors as the patient's age, weight, height, sex, general medical condition and previous medical history.

In some embodiments of the invention, the method comprises administration of multiple doses of the compounds of the subject invention. The method may comprise administration of 1, 2, 3, 4, 5, 6, 7, 8, 9, 10, 15, 20, 25, 30, 35, 40, 50, 75, 100, 150, 200, 250, 300, 350, 400, 450, 500, 600, 700, 800, 900, 1000 or more therapeutically effective doses of a composition comprising the compounds of the subject invention as described herein. In some embodiments, doses are administered over the course of 1 day, 2 days, 3 days, 4 days, 5 days, 6 days, 7 days, 10 days, 14 days, 21 days, 30 days, 2 months, 3 months, 6 months, 1 year, 2 years, 3 years, 4 years, 5 years, 6 years, 7 years, 8 years, 9 years, 10 years, or more than 10 years. The frequency and duration of administration of multiple doses of the compositions is such as to inhibit or delay the initiation of myopia. Moreover, treatment of a subject with a therapeutically effective amount of the compounds of the invention can include a single treatment or can include a series of treatments. It will also be appreciated that the effective dosage of a compound used for treatment may increase or decrease over the course of a particular treatment. Changes in dosage may result and become apparent from the results of diagnostic methods for detecting myopia known in the art and described herein. In some embodiments of the invention, the method comprises administration of the compounds at a single time per day or several times per day, including but not limiting to 2 times per day, 3 times per day, and 4 times per day.

As used herein, the term "myopia" refers to a subject having at least one eye with an Spherical Equivalence (SE) value less than or equal to -0.5 D, such as, for example, -1.0 D, -2.0 D.

As used herein, the term "pre-myopia" refers to a subject having at least one eye with an SE value of about -0.49 D to about 1.00 D.

As used herein, The term "mild myopia" refers to a subject having at least one eye with an SE value of about -0.50 D to -1.50 D.

As used herein, The term "moderate myopia" refers to a subject having at least one eye with an SE value of about -3.00 D to -6.00 D.

The term "high myopia" refers to a subject having at least one eye with an SE value of about -6.0 D or less, such as, for example -7.0 D or -8.0 D.

As used herein, the term "drop" refers to a unit of measure of volume, which is equal to the amount dispensed as one drop from a dropper or drip chamber. In certain embodiments, a drop can contain about 20 µl to about 100 µl, about 30 µl to 70 µl, or about 50 µl of liquid.

As used herein, the phrase "accommodation amplitude", refers to the ability of the eye to focus on near objects. A decrease in accommodation amplitude may reduce near vision such that subjects affected need bifocal or progressive glasses to read or see close objects.

### Compounds

In preferred embodiments, the compositions and methods according to the subject invention utilize atropine, including, for example, atropine salts (e.g., atropine sulfate).

All medications can be manufactured by Aseptic Innovative Medicine Co., Ltd. (Taoyuan City, Taiwan) The drugs can be a clear, colorless, and sterile solution. The solutions can be provided in 0.5 mL low density polyethylene (LDPE) ampoules, however, other storage and/or administration containers can be envisioned. The compositions can be stored below 25°C and avoid direct light.

Atropine may be added to compositions at concentrations of about 0.0001 to about 5% by weight (wt %), preferably about 0.01 to about 0.5 wt%, and most preferably about 0.025% to about 0.05 wt%. In another embodiment, atropine can be in combination with an acceptable carrier and/or excipient, in that atropine may be presented at concentrations of about 0.0001 to about 5% (v/v), preferably, about 0.01 to about 0.5% (v/v), more preferably, about 0.025 to about 0.05% (v/v), or, most preferably, greater than 0.025 to about 0.05% (v/v). In certain embodiments, atropine can be present in the composition are a concentration of about 0.010%, about 0.011%, about 0.012%, about 0.013%, about 0.014%, about 0.015%, about 0.016%, about 0.017%, about 0.018%, about 0.019%, about 0.020%, about 0.021%, about 0.022%, about 0.023%, about 0.024%, about 0.025%, about 0.026%, about 0.027%, about 0.028%, about 0.029%, about 0.03%, about 0.031%, about 0.032%, about 0.033%, about 0.034%, about 0.035%, about 0.036%, about 0.037%, about 0.038%, about 0.039%, about 0.04%, about 0.041%, about 0.042%, about 0.043%, about 0.044%, about 0.045%, about 0.046%, about 0.047%, about 0.048%, about 0.049%, about 0.05%, about 0.051%, about 0.05%, about 0.052%, about 0.053%, about 0.053%, about 0.054%, about 0.055%, about 0.056%, about 0.057%, about 0.058%, about 0.059%, or about 0.06%.

In certain embodiments, the compositions are preferably administered to the eye, including, for example, ophthalmic administration, conjunctival administration, intracorneal administration, intraocular administration, intravitreal administration, or retrobulbar administration.

In one embodiment, the orally consumable product is a, syrup, emulsion, or liquid suspension containing a desired orally deliverable substance.

The subject composition can further comprise one or more pharmaceutically acceptable carriers, and/or excipients, and can be formulated into preparations, for example, semi-solid, liquid, or gaseous forms, such as ointments, solutions, suppositories, and injections.

The term "pharmaceutically acceptable" as used herein means compatible with the other ingredients of a pharmaceutical composition and not deleterious to the recipient thereof.

Carriers and/or excipients according the subject invention can include any and all solvents, diluents, buffers (such as, e.g., neutral buffered saline, phosphate buffered saline, or optionally Tris-HCl, acetate or phosphate buffers), oil-in-water or water-in-oil emulsions, aqueous compositions with or without inclusion of organic co-solvents suitable for use, e.g., IV solubilizers (e.g., Polysorbate 65, Polysorbate 80), colloids, dispersion media, vehicles, fillers, chelating agents (e.g., EDTA or glutathione), amino acids (e.g., glycine), proteins, disintegrants, binders, lubricants, wetting agents, emulsifiers, sweeteners, colorants, flavorings, aromatizers, thickeners (e.g. carbomer, gelatin, or sodium alginate), coatings, preservatives (e.g., Thimerosal, benzyl alcohol, polyquaterium), antioxidants (e.g., ascorbic acid, sodium metabisulfite), tonicity controlling agents, absorption delaying agents, adjuvants, bulking agents (e.g., lactose, mannitol) and the like. The use of carriers and/or excipients in the field of drugs and supplements is well known. Except for any conventional media or agent that is incompatible with the target health-promoting substance or with the composition, carrier or excipient use in the subject compositions may be contemplated. In certain embodiments, the compositions can comprise a polymer, such as, for example, Hypromellose (HPMC)-606, a salt, such as, for example, sodium phosphate monobasic, sodium chloride, sodium borate, and an acid, such as, for example, boric acid or hydrochloric acid.

In certain embodiments, medications can be formulated as:
i) 0.01%. Each mL contains: atropine sulfate 0.1 mg. Excipients: HPMC-606 5.0 mg, sodium chloride 8.0 mg, boric acid 1.0 mg, sodium borate 0.1mg and hydrochloric acid for pH adjustment to a pH of about 3.5 to about 6.0; or
ii) 0.05%. Each mL contains: atropine sulfate 0.5 mg. Excipients: sodium phosphate monobasic•2H₂O 0.4 mg, sodium chloride 8.5 mg, hydrochloric acid for pH adjustment to a pH of about 3.5 to about 6.0.

In one embodiment, the compositions of the subject invention can be formulated for administration via injection, for example, as a solution or suspension. The solution or suspension can comprise suitable non-toxic, parenterally-acceptable diluents or solvents, such as mannitol, 1,3-butanediol, water, Ringer's solution, or isotonic sodium chloride solution, or suitable dispersing or wetting and suspending agents, such as sterile, non-irritant, fixed oils, including synthetic mono- or diglycerides, and fatty acids, including oleic acid. One illustrative example of a carrier for intravenous use includes a mixture of 10% USP ethanol, 40% USP propylene glycol or polyethylene glycol 600 and the balance USP Water for Injection (WFI). Other illustrative carriers for intravenous use include 10% USP ethanol and USP WFI; 0.01-0.1% triethanolamine in USP WFI; or 0.01-0.2% dipalmitoyl diphosphatidylcholine in USP WFI; and 1-10% squalene or parenteral vegetable oil-in-water emulsion. Water or saline solutions and aqueous dextrose and glycerol solutions may be preferably employed as carriers, particularly for injectable solutions. Illustrative examples of carriers for subcutaneous or intramuscular use include phosphate buffered saline (PBS) solution, 5% dextrose in WFI and 0.01-0.1% triethanolamine in 5% dextrose or 0.9% sodium chloride in USP WFI, or a 1 to 2 or 1 to 4 mixture of 10% USP ethanol, 40% propylene glycol and the balance an acceptable isotonic solution such as 5% dextrose or 0.9% sodium chloride; or 0.01-0.2% dipalmitoyl diphosphatidylcholine in USP WFI and 1 to 10% squalene or parenteral vegetable oil-in-water emulsions.

### Methods of Using Compounds of the Subject Invention

In certain embodiments, methods of administering to a subject a composition comprising atropine are provided. In certain embodiments, the subjects are not myopic before the atropine treatment; including, for example, the subject has a positive SE value or a negative value of 0 to -0.5 D, such as, for example -0.3 D. In certain embodiments, the subject has an SE value of about +1.00 D to -0.49 D, about 0.75 D to about -0.49 D, or about +1.00 D to about 0 D.

In certain embodiments, the subject can be of any age. In preferred embodiments, the subject is at least about 4 years old. In preferred embodiments, the subject is about 4 to about 9 years old. In some cases, the subject has no history of ophthalmic diseases other than refractive errors. In some cases, the subject has an astigmatism of less than about 1.00 D, anisometropia of less than about 2.0 D, or any combination thereof. In some cases, the subject has at least one parent who is myopic, including, for example, at least one parent whose SE is less than or equal to -3.00 D.

In certain embodiments, at least one method can be used to determine the ocular health of a subjects before, during, or after administration of the compositions comprising atropine. Non-limiting exemplary methods include determining the best corrected visual acuity for near and distant (EDTRS) charts, near aided visual acuity, ocular alignment using a covered and uncovered test, extraocular movement, pupil size, intraocular pressure by non-contact tonometry (NCT), accommodation amplitude by measurement of the Royal Air Force near point rule, and/or cycloplegic autorefraction. In some cases, two, three or all of the aforementioned methods are performed to evaluate the patient's ocular health, including assessing the degree of myopia by determining SE values.

In certain embodiments, effects of the compositions on the subject can be determined. Atropine's effect on preventing or delaying the onset of myopia can be assessed by examining the eye during periodic visits during treatment and, optionally, after treatment and determining the timing when the patient first becomes myopic, i.e., when the patient's SE of the subject is about -0.5 D. The timing is then compared with the timing of the onset of myopia in a control group that does not receive an administration of a composition comprising atropine but instead receives vehicle placebo eye drops. The control subject is typically of a similar age and ethnic background to the patient treated with atropine. The subjects in the control group may also have an ocular health status comparable to that of the individuals in the treatment group before the beginning of atropine treatment. In preferred embodiments, the individuals from the control group are pre-myopic and between ages of about 4 and about 9.

In certain embodiments, the subject can be monitored for ocular health at the beginning of the treatment and during periodic examinations during and/or after treatment. The subjects can be monitored, such as, for example, every one, two, three, four, five, six, seven, or eight months during and/or after the treatment period. In some cases, patients are examined for ocular health, including the degree of myopia (e.g., axial distance and/or SE value) every about 4 to about 6 months after treatment begins.

In certain embodiments, the compositions comprising atropine can be administered to a subject to inhibit or delay the onset of myopia by administration to the eye. In some embodiments, doses are administered over the course of about 30 days, about 2 months, about 3 months, about 6 months, about 9 months, about 1 year, about 2 years, about 3 years, about 4 years, about 5 years, about 6 years, about 7 years, about 8 years, about 9 years, about 10 years, or more than 10 years.

In some embodiments, treating the patient with the composition disclosed herein can delay the onset of myopia for at least about 1 month, about 2 months, about 3 months, about 4 months, about 5 months, about 6 months, about 12 months, about 18 months, about two years, about three years, about 4 years, about five years, about six years, about 7 years, or about eight years as compared to controls. For purpose of this disclosure, controls refer to individuals who are not treated with the atropine composition disclosed herein. In preferred embodiments, the individuals in the control group are from the same age group and at the same pre-myopic as the individuals who are treated with the compositions disclosed herein.

In certain embodiments, the subject composition can be delivered to the subject via a topical route, formulated as solutions, suspensions, emulsions, gels, ointments, pastes, etc. In preferred embodiments, the composition can be a liquid solution to be applied to the eye of a subject (i.e., ophthalmic administration). In certain embodiments, at least 1, 2, 3, 4, 5, 6, 7, 8, 9, or 10 drops can be administered to a subject. In certain embodiments, one drop can have a volume of about 20 µL to about 100 µL, about 25 µL to about 75 µL, or about 50 µL.

In certain embodiments, the composition can be administered at any time in a day. In certain embodiments, the composition can be administered to a subject once, twice, thrice or more times per day. In certain embodiments, the composition can be administered every two days, every three days, or every four days. In certain embodiments, each administration can occur at a consistent time period, volume, and concentration. In certain embodiments, the time period between administrations can increase or decrease during the treatment period. In certain embodiments, the volume of the administrations can increase or decrease during the treatment period. In certain embodiments, the concentration of the compositions administered can increase or decrease during the treatment period.

In certain embodiments, the treatment period can occur for at least about one year, about two years, about 3 years, about four years, about five years, about six years, about seven years, about eight years, or about ten years. In certain embodiments, the composition can be administered 6 months to about 1 year, 18 months to 2 years, 1 year to 2 years, 16 to 32 months, 24 to 48 months, 32 to 48 months, 32 to 52 months, 48 to 52 months, 48 to 64 months, 52 to 64 months, 52 to 72 months, 64 to 72 months, 64 to 80 months, 72 to 80 months, 72 to 88 months, 80 to 88 months, 80 to 96 months, 88 to 96 months, and 96 to 104 months. Suitable periods of administration also include 6, 7, 8, 9, 10, 11, 12, 13, 14, 15, 16, 17, 18, 19, 20, 24, 28, 30, 32, 36, 42, 48, and 56 months. Generally administration of the composition should be continued as long as clinically significant myopia inhibition or delaying of myopia is observed.

In some embodiments, administration of an atropine composition is not continuous and can be stopped for one or more periods of time, followed by one or more periods of time where administration resumes. Suitable periods where administration stops include 5 to 9 months, 5 to 16 months, 9 to 16 months, 16 to 24 months, 16 to 32 months, 24 to 32 months, 24 to 48 months, 32 to 48 months, 32 to 52 months, 48 to 52 months, 48 to 64 months, 52 to 64 months, 52 to 72 months, 64 to 72 months, 64 to 80 months, 72 to 80 months, 72 to 88 months, 80 to 88 months, 80 to 96 months, 88 to 96 months, and 96 to 100 months. Suitable periods where administration stops also include 5, 6, 7, 8, 9, 10, 11, 12, 13, 14, 15, 16, 17, 18, 19, 20, 24, 25, 30, 32, 35, 40, 45, 48 50, 52, 55, 60, 64, 65, 68, 70, 72, 75, 80, 85, 88 90, 95, 96, and 100 months.

In certain embodiments, atropine can be used in combination with other active agents known to be useful for inhibiting or delaying the onset of myopia.

In certain embodiments, the mean change in refraction following the start of administration of the composition can be reduced by at least about 10%, about 20%, about 30%, about 31%, about 35%, about 36%, about 37%, about 40%, about 50%, about 60%, about 70%, about 75%, about 78%, about 79%, or about 80% over a period of at least about two weeks, about one month, about two months, about six months, about one year, about two years, about three years, about four years, about five years, about six years, about seven years, or about eight years from the initiation of the treatment as compared to controls.

In some embodiments, the mean change in of axial length following the start of administration of the composition can be reduced by at least about 5%, about 10%, about 15%, about 17%, about 18%, about 20%, or about 25%, about 30%, about 31%, about 35%, about 36%, about 37%, or about 40% over a period of at least about two weeks, about one month, about two months, about six months, about one year, about two years, about three years, about four years, about five years, about six years, about seven years, or about eight years from the initiation of the treatment as compared to controls.

In certain embodiments, the incidence of myopia following the start of administration of the composition can be reduced by at least about 10%, about 20%, about 25%, about 26%, about 30%, about 35%, about 36%, about 37%, about 40%, about 50%, about 60%, about 63%, about 64% about 70%, or about 75% over a period of at least about two weeks, about one month, about two months, about six months, about one year, about two years, about three years, about four years, about five years, about six years, about seven years, or about eight years from the initiation of the treatment as compared to controls.

In certain embodiments, changes in accommodation amplitude during the treatment period can be small, in which accommodation amplitude in a subject treated with atropine and a subject in a placebo group can be in a range of about 1 D to about 20 D or about 5 D to about 15 D in which the accommodation amplitude varies by less than about 6 D, about 5 D, about 4 D, about 3 D, about 2 D, about 1 D, about 0.5 D, about 0.3 D, about 0.2 D, or about 0.1 D between a subject treated with atropine and a subject treated with a placebo.

In certain embodiments, changes in mesopic pupil size during the treatment period can be small, in which mesopic pupil size in a subject treated with atropine and a subject in a placebo group can be in a range of about 1 mm to about 10 mm or about 5 mm to about 8 mm in which the mesopic pupil sizes varies by less than about 3 mm, about 2 mm, about 1 mm, about 0.5 mm, about 0.3 mm, about 0.2 mm, or about 0.1 mm between a subject treated with atropine and a subject treated with a placebo.

In certain embodiments, changes in photopic pupil size during the treatment period can be small, in which photopic pupil size in a subject treated with atropine and a subject in a placebo group can be in a range of about 1 mm to about 10 mm or about 5 mm to about 8 mm in which the photopic pupil sizes varies by less than about 2 mm, about 1 mm, about 0.5 mm, about 0.3 mm, about 0.2 mm, or about 0.1 mm between a subject treated with atropine and a subject treated with a placebo.

In certain embodiments, changes in distant visual acuity during the treatment period can be small, in which distant visual acuity in a subject treated with atropine and a subject in a placebo group can be in a range of about -0.01 to about -0.05 logMAR for a subject treated with atropine compositions or about -0.01 to about -0.06 logMAR for a subject treated with a placebo.

In certain embodiments, changes in near visual acuity can be small, in which near visual acuity in a subject treated with atropine and a subject in a placebo group can be in a range of about 0.001% to about 0.1% or about 0.01% to about 0.07%.

### MATERIALS AND METHODS

### Study design

The study is a double blinded randomized clinical trial. Eligible children will be randomized into groups: treatment group: 0.01% atropine both eyes once daily; treatment group: 0.05% atropine both eyes once daily; and a placebo group: eye drops both eyes once daily.

For the first two years, all the groups remained unchanged.

After two years, children from the placebo group will remain in the placebo group until they have onset of myopia with a spherical equivalence (SE) ≤ -0.5 Diopter (D). That is, they can be switched to the treatment group administering 0.05% atropine once they have onset of myopia (SE≤-0.5D). Children from treatment group administering 0.01% atropine will remain in the group until they have onset of myopia with SE ≤ -0.5 D. That is, they can be switched to treatment group administering 0.05% atropine year once they have onset of myopia (SE≤-0.5D). Treatment group administering 0.05% atropine will remain in the same group throughout the whole study period.

Eligible subjects are children aged 4 to 9 with a SE of 0 D to +1.0 D, astigmatism of < 1.00 D; anisometropia of < 2.0 D; at least one parent whose SE is ≤ -3.00 D; and informed parental consent. Subjects are excluded if he/she has ophthalmic diseases other than refractive errors, or previous use of treatment of atropine, or an allergy or intolerance to atropine, and/or an inability to attend regular follow up assessment.

The age limit of 4-9 years old is set due to the reasons below. Children younger than 4 years old can be very difficult for accurate cycloplegic examination and biometry measurement. Also, early onset myopes tend to be a fast progressor. Therefore we do not include children older than 9 years.

Compared to having both non-myopic parents, having one moderate myopic parent confer a 2-fold increased risk, and having both highly myopic parents confers a 12-fold increased risk, for children to develop myopia. Therefore, children with at least one parent moderate myopic will be recruited.

To calculate the required number of study subjects, we took the estimated myopia onset rate for 0.05%, and 0.01% atropine and placebo groups to be and 6.6%, 14.6%, 20% respectively. The 0.05% atropine group should have the smallest number of myopic onset, thus a minimum of 5 to 10 myopia children should be observed. To detect a difference among treatment groups, a sample size of 375 subjects (125 per group) could achieve 90% power at a 0.05 significance level. By factoring in an attrition rate of 20%, a sample size of 474 subjects (158 per group) would be needed.

### Methods of Administration

This is a double-blinded randomized control trial that lasts for four years. All participating children will be randomized into one of the following groups in 1:1:1 ratio for treatment group: 0.01% atropine both eyes once daily; treatment group: 0.05% atropine both eyes once daily; and a placebo group: eye drops both eyes once daily.

Children are offered photochromatic glasses if they experienced glare or their parents are worried about excessive light exposure, or progressive glasses if children experienced difficulty with near vision. The treatment can be continued for four years.

### Initial Visit

This is a double-blinded randomized control trial, last for four years.

All participating children will be randomized into one of the following groups in 1:1:1 ratio:
Initial Visit
   Aim:
   - To obtain informed consent
   - To confirm the eligibility
   - To obtain baseline data
History
   - Ocular history: history of ocular disease and operation
   - Refraction
   - Systemic review
Examination
   - Best corrected visual acuity for near and distant (EDTRS) charts
   - Near aided visual acuity
   - Cover and uncover test to test ocular alignment
   - Extraocular movement
   - Pupil size
   - Intraocular pressure by non-contact tonometry (NCT)
   - Measurement of accommodation amplitude by Royal Air Force near point rule
   - Instillation of cycloplegic agent:
      a. Wash hands.
      b. Tilt patient's head back and make a pouch with the lower cul-de-sac. Install one (1) drop of Mydrin-P and cyclopentolate eye drop. Releases pouch and apply pressure on the inner canthus for a period of ten seconds to minimize systemic absorption. Repeat for the other eye.
      c. After a ten (10) minute interval, repeat step 2.
      d. After a further ten (10) minute interval, repeat step 2 such that a total of three (3) drops of Mydrin-P and cyclopentolate are instilled into each eye.
      e. Perform auto refraction thirty (30) minutes after the last set of drops.
   - Cycloplegic autorefraction
      a. Turn the power switch ON. The target rings will appear on the TV monitor.
      b. Set the instrument on the R/K mode. It will be automatically set if this was the mode when the machine was turned off.
      c. Instruct the patient to place his chin on the chin rest and forehead on the headrest gently. Adjust the machine height so that the patient's eye level will meet the eye level indicator when looking straight ahead.
      d. Place the patient's eye in the middle of the TV monitor by manipulating the joystick. Focus on the blinking light by manipulating the joystick back and forth and make sure it is inside the inner target ring.
      e. Instruct the patient to look at the picture and to hold his/her eyes open wide. He/she should refrain from blinking during the measurement procedure.
      f. Press the start button.
      g. Repeat ( steps e&f) for at least 3 readings.

      - Choroid thickness measured by DRI Swept Source Optical Coherence Tomography.
      - Slit-lamp examination & binocular indirect ophthalmoscopy examination
      - Ocular biometry by IOL master
      - Perform six measurements of total axial length, anterior chamber length and corneal curvatures at least 3 measurements are identical or almost identical. Discord outline. Print results.
   - Questionnaire
      - Parental history of myopia
      - Parental educational level
      - Self-reported visual function questionnaire
      - Questionnaires on amount of near works and out-door activities.

### Randomization

Block randomization will be used to assign consecutive cases into either treatment arm in the proposed RCT. The block size could be 4, 6 or 8 (the actual block size will be kept confidential to all of the investigators and participating staffs except for the statistician who design the randomization strategies). Since age has been shown to be a major factor that associated with the onset of myopia, we will randomize eligible subjects in 8 strata separately (i.e. 1) age 4-6 female in +1.00D ≤ SE < +0.50D, 2) age 4-6 male in +1.00D ≤ SE < +0.50D, 3) age 4-6 female in +0.50D ≤ SE ≤ 0.00D, 4) age 4-6 male in +0.50D ≤SE ≤ 0.00D, 5) age 7-9 female in +1.00D ≤ SE < +0.50D, 6) age 7-9 male in +1.00D ≤ SE < +0.50D, 7) age 7-9 female in +0.50D ≤ SE ≤ 0.00D, and 8) age 7-9 male in +0.50D ≤ SE ≤ 0.00D). Randomization table will be generated and kept confidential by a statistician independently. Only the staff who is designated as the person to obtain the randomization code can get access to the table and assign treatment arms for each study subject. The investigator, the patient and the staff who conducts the follow-up examinations will be kept unaware of the randomization information. In the study design, we have taken into account the possible effect of covariates on the estimated sample size.

### Blinding method

The assignment of groups will not be disclosed to the subjects (including their parents) and investigators. All types of eye drops (atropine 0.01%, 0.05% and lubricant) will be contained in the same type of bottle. All the optometrists responsible for assessing the study outcomes will be blinded to the treatment given to each of the children. The statistician will also be blinded to the assigned treatment for each child.

### Visit 2 at 2 weeks after the treatment (Baseline 2 + assessment of tolerance)

### Examination as in visit 1

Cycloplegic refraction at visit 2 will be used as baseline 2 for future comparison of myopia progression if any, as hyperopic shift may occur after commencing atropine eye drops. Children will be offered photochromatic glasses if they experience glare or their parents are worried about excessive light exposure, or progressive glasses if children experience difficulty with near vision.

### Visits 3-8 (Treatment follow-up visits, every 4 months)

- To assess study outcomes
- To assess compliance by history, amount of drug used
- To assess complications
- To update glasses prescription if SE change > 0.75D
- Examination as visit 1.

### Visits 9-12 (Treatment follow-up visits, every 6 months)

The placebo group will be switched to 0.05% atropine group during the third year when SE ≤ -0.5D. Subject will be kept in the placebo group when SE remains > -0.5D. 0.01% atropine group will be switched to 0.05% atropine group during the third year when SE ≤ -0.5D. Subject will be kept in the 0.01% atropine group when SE remains > -0.5D.

### Unscheduled visit

The children are advised to come back for assessment when any problems with the treatments arise, such as deterioration of vision, treatment complications. The children will have detailed examination. New prescription will be given when indicated. When there is severe side effect associated with the treatment, the treatment will be stopped. The children will be asked to come back at the original schedule after the unscheduled visit

### Visit schedule

| | |
|---|---|
| Visit 1 | Day 0 (Baseline 1) |
| Visit 2 | Week 2 (Baseline 2 + assessment of tolerance) |
| Visit 3 | Month 4 (Treatment follow-up visits) |
| Visit 4 | Month 8 (Treatment follow-up visits) |
| Visit 5 | Month 12 (Treatment follow-up visits) |
| Visit 6 | Month 16 (Treatment follow-up visits) |
| Visit 7 | Month 20 (Treatment follow-up visits) |
| Visit 8 | Month 24 (Treatment follow-up visits) |
| Visit 9 | Month 30 (Treatment follow-up visits) |
| Visit 10 | Month 36 (Treatment follow-up visits) |
| Visit 11 | Month 42 (Treatment follow-up visits) |
| Visit 12 | Month 48 (Treatment follow-up visits) |

### Study outcome & assessment

Myopia is defined as a spherical equivalent refractive error of at least -0.50D, based on the Refractive Error Study in Children. The Proportion of onset of myopia and proportion of fast myopia progressor in each group can be determined to establish efficacy. Myopic progression can be measured by change in spherical equivalent refraction (SER). Cycloplegic refraction is assessed by using an auto-refractometer. Eyeball growth is measured by change in axial length, using the IOL master.

### Analysis

Intention-to-treat analysis will be employed. The proportion of onset of myopia and proportion of fast myopic progressor in each treatment group are the primary outcome measures. Change from baseline SER and axial length in the treatment group and the control group are the secondary outcome measures. These will be analyzed by multiple regression models. Baseline measurements, treatment group, parental history of myopia, age, and their interaction will be used as factors in the model. The analysis of safety measures made on categorical or frequency scales will be based on chi-square statistics. Analysis of Variance (ANOVA) model will be built up to identify the treatment effect, period effect, carry-over effect and their interaction

### Adverse effects & patient withdrawal / exit from study

Atropine is known to occasionally can cause side-effects. Systemic effects include, for example, tachycardia, respiratory stress, allergic dermatitis. Ocular effects include increase in intraocular pressure (IOP), phototoxicity and early presbyopia.

These potential side-effects can be closely monitored, so can be the physical integrity of the cornea, lens and retina. They can be examined with direct examination, serial photography and specular microscopy. IOP is measured with non-contact tonometry (NCT) and/or applanation. The data then can be used to project the proportion of the patient population (4-9 year-olds) that may become sensitive to topical atropine.

All patents, patent applications, provisional applications, and publications referred to or cited herein are incorporated by reference in their entirety, including all figures and tables, to the extent they are not inconsistent with the explicit teachings of this specification.

Following are examples that illustrate procedures for practicing the invention. These examples should not be construed as limiting. All percentages are by weight and all solvent mixture proportions are by volume unless otherwise noted.

### EXAMPLE 1-EFFECTIVENESS

The mean and standard deviation of change in SE for 1 year in 429 children: -0.55 ± 0.60 D, -0.38 ± 0.53, and -0.12 ± 0.48 D, for placebo, 0.01%, and 0.05% atropine daily, respectively (**FIG**. **1**). Mean and SD of change in axial length for 1 year in our study: 0.41 ± 0.25, 0.34 ± 0.21, and 0.26 ± 0.19 mm, respectively (**FIG**. **2**). The 0.05% atropine can reduce the change in refraction by 78.2%, reduce the axial elongation by 36.6%, and reduce the myopia incidence rate by 63%; whereas the 0.01% atropine can reduce the change in refraction by 30.9%, reduce the axial elongation by 17.1 %, and reduce the myopia incident rate by 25.9% (**FIG**. **3**).

### EXAMPLE 2-SIDE EFFECTS

In our series of atropine studies, the 0.05% atropine was well tolerated by all the children in pupil dilation, accommodation loss, near vision, and best-corrected distant vision. The vision-related quality of life was not affected. Changes in accommodation amplitude were small. Accommodation amplitude in both 0.05% and 0.01% atropine groups were comparable with the placebo group, 13.35 ± 2.91, 13.63 ± 2.61 and 13.35 ± 2.74 D in the 0.05% atropine group, and the 0.01% atropine group, and the placebo group, respectively (**Table 1**). The changes in pupil size was comparable in both 0.05% and 0.01% atropine. Mesopic pupil sizes were 6.37 ± 0.73, 6.32 ± 0.82 and 6.57 ± 0.79 mm in 0.05% atropine, 0.01% atropine and placebo groups, respectively; while photopic pupil sizes were 3.53 ± 0.59, 3.65 ± 0.70, and 3.69 ± 0.66 mm, in 0.05% atropine, 0.01% atropine, and placebo groups, respectively. Both distant BCVA and near visual acuity in both 0.05% and 0.01% atropine were not affected significantly. Distant visual acuity: 0.03 ± 0.09, 0.02 ± 0.08, and 0.03 ± 0.08, for 0.05% and 0.01% atropine and placebo group, respectively, while near visual acuity: 0.05 ± 0.11, 0.03 ± 0.10, and 0.02 ± 0.09, for 0.05% and 0.01% atropine and placebo groups, respectively. The 0.05% atropine composition is safe and more effective than 0.01% atropine composition in inhibiting the onset of myopia.

**Table 1: Side effects over 2 years**

| | 0.05% Atropine | | | | 0.01% Atropine | | | | Placebo | | | |
|---|---|---|---|---|---|---|---|---|---|---|---|---|
| | First year | | Second year | | First year | | Second year | | First year | | Second year | |
| Photochromatic glasses needed | 0 | 0.00% | 0 | 0.00% | 1 | 0.72% | 2 | 1.65% | 0 | 0.00% | 2 | 1.75% |
| Progressive 0 glasses needed | | 0.00% | 0 | 0.00% | 0 | 0.00% | 1 | 0.00% | 1 | 0.79% | 0 | 0.00% |
| Photophobia | 28 | 20.74% | 15 | 13.16% | 29 | 20.86% | 23 | 19.01% | 13 | 10.24% | 14 | 12.28% |
| Allergic | 5 conjunctivitis | 3.70% | 5 | 4.39% | 7 | 5.04% | 3 | 2.48% | 8 | 6.30% | 2 | 1.75% |

It should be understood that the examples and embodiments described herein are for illustrative purposes only and that various modifications or changes in light thereof will be suggested to persons skilled in the art and are to be included within the spirit and purview of this application and the scope of the appended claims. In addition, any elements or limitations of any invention or embodiment thereof disclosed herein can be combined with any and/or all other elements or limitations (individually or in any combination) or any other invention or embodiment thereof disclosed herein, and all such combinations are contemplated with the scope of the invention without limitation thereto.

### REFERENCES

1. Fan DS, Lam DS, Lam RF, Lau JT, Chong KS, Cheung EY, Lai RY, Chew SJ: Prevalence, incidence, and progression of myopia of school children in Hong Kong. Invest Ophthalmol Vis Sci 2004, 45(4):1071-1075.
2. Lam CS, Lam CH, Cheng SC, Chan LY: Prevalence of myopia among Hong Kong Chinese schoolchildren: changes over two decades. Ophthalmic Physiol Opt 2012, 32(1):17-24.
3. Fan DS, Lai C, Lau HH, Cheung EY, Lam DS: Change in vision disorders among Hong Kong preschoolers in 10 years. Clin Exp Ophthalmol 2011, 39(5):398-403.
4. Resnikoff S, Pascolini D, Mariotti SP, Pokharel GP: Global magnitude of visual impairment caused by uncorrected refractive errors in 2004. Bull World Health Organ 2008, 86(1):63-70.
5. Holden BA, Fricke TR, Wilson DA, Jong M, Naidoo KS, Sankaridurg P, Wong TY, Naduvilath TJ, Resnikoff S: Global Prevalence of Myopia and High Myopia and Temporal Trends from 2000 through 2050. Ophthalmology 2016, 123(5):1036-1042.
6. Kwok MK, Yam JC, See ML, AL. Y: An update on the interventions and strategies in prevention of myopia progression. Hong Kong Practitioner 2018, 2013(35):91-96
7. Release of China's first oversight report on quality of compulsory education [http://en.moe.gov.cn/News/Top_News/201808/t20180801_344002.html]
8. Xi Jinping: Take care of your children's eyes and let them have a bright future. [https://mp.weixin.qq.com/s/GttINBOhdkApTVsZmO7fqQ]
9. Ministry of Education: Implementation Plan for the Prevention and Control of Children and Adolescents' Myopia [http://www.moe.edu.cn/srcsite/A17/moe_943/s3285/201808/t20180830_346672.html]
10. Rose KA, Morgan IG, Ip J, Kifley A, Huynh S, Smith W, Mitchell P: Outdoor activity reduces the prevalence of myopia in children. Ophthalmology 2008, 115(8):1279-1285.
11. He M, Xiang F, Zeng Y, Mai J, Chen Q, Zhang J, Smith W, Rose K, Morgan IG: Effect of Time Spent Outdoors at School on the Development of Myopia Among Children in China: A Randomized Clinical Trial. JAMA 2015, 314(11):1142-1148.
12. Wu PC, Chen CT, Lin KK, Sun CC, Kuo CN, Huang HM, Poon YC, Yang ML, Chen CY, Huang JC, Wu PC, Yang IH, Yu HJ, Fang PC, Tsai CL, Chiou ST, Yang YH: Myopia Prevention and Outdoor Light Intensity in a School-Based Cluster Randomized Trial. Ophthalmology 2018, 125(8):1239-1250.
13. Leung JT, Brown B: Progression of myopia in Hong Kong Chinese schoolchildren is slowed by wearing progressive lenses. Optom Vis Sci 1999, 76(6):346-354.
14. Gwiazda J, Hyman L, Hussein M, Everett D, Norton TT, Kurtz D, Leske MC, Manny R, Marsh-Tootle W, Scheiman M: A randomized clinical trial of progressive addition lenses versus single vision lenses on the progression of myopia in children. Invest Ophthalmol Vis Sci 2003, 44(4):1492-1500.
15. Correction of Myopia Evaluation Trial 2 Study Group for the Pediatric Eye Disease Investigator G: Progressive-addition lenses versus single-vision lenses for slowing progression of myopia in children with high accommodative lag and near esophoria. Invest Ophthalmol Vis Sci 2011, 52(5):2749-2757.
16. Fulk GW, Cyert LA, Parker DE: A randomized trial of the effect of single-vision vs. bifocal lenses on myopia progression in children with esophoria. Optom Vis Sci 2000, 77(8):395-401.
17. Cho P, Cheung SW: Retardation of myopia in Orthokeratology (ROMIO) study: a 2-year randomized clinical trial. Invest Ophthalmol Vis Sci 2012, 53(11):7077-7085.
18. Hiraoka T, Kakita T, Okamoto F, Takahashi H, Oshika T: Long-term effect of overnight orthokeratology on axial length elongation in childhood myopia: a 5-year follow-up study. Invest Ophthalmol Vis Sci 2012, 53(7):3913-3919.
19. Kanda H, Oshika T, Hiraoka T, Hasebe S, Ohno-Matsui K, Ishiko S, Hieda O, Torii H, Varnas SR, Fujikado T: Effect of spectacle lenses designed to reduce relative peripheral hyperopia on myopia progression in Japanese children: a 2-year multicenter randomized controlled trial. Jpn J Ophthalmol 2018, 62(5):537-543.
20. Fan DS, Lam DS, Chan CK, Fan AH, Cheung EY, Rao SK: Topical atropine in retarding myopic progression and axial length growth in children with moderate to severe myopia: a pilot study. Jpn J Ophthalmol 2007, 51(1):27-33.
21. Chua WH, Balakrishnan V, Chan YH, Tong L, Ling Y, Quah BL, Tan D: Atropine for the treatment of childhood myopia. Ophthalmology 2006, 113(12):2285-2291.
22. Chia A, Chua WH, Cheung YB, Wong WL, Lingham A, Fong A, Tan D: Atropine for the treatment of childhood myopia: safety and efficacy of 0.5%, 0.1%, and 0.01% doses (Atropine for the Treatment of Myopia 2). Ophthalmology 2012, 119(2):347-354.
23. Chia A, Lu QS, Tan D: Five-Year Clinical Trial on Atropine for the Treatment of Myopia 2: Myopia Control with Atropine 0.01% Eyedrops. Ophthalmology 2016, 123(2):391-399.
24. Yam JC, Jiang Y, Tang SM, Law AKP, Chan JJ, Wong E, Ko ST, Young AL, Tham CC, Chen LJ, Pang CP: Low-Concentration Atropine for Myopia Progression (LAMP) Study. Ophthalmology 2018.
25. Walline JJ, Lindsley K, Vedula SS, Cotter SA, Mutti DO, Twelker JD: Interventions to slow progression of myopia in children. Cochrane Database Syst Rev 2011(12):CD004916.
26. Huang J, Wen D, Wang Q, McAlinden C, Flitcroft I, Chen H, Saw SM, Chen H, Bao F, Zhao Y, Hu L, Li X, Gao R, Lu W, Du Y, Jinag Z, Yu A, Lian H, Jiang Q, Yu Y, Qu J: Efficacy Comparison of 16 Interventions for Myopia Control in Children: A Network Meta-analysis. Ophthalmology 2016, 123(4):697-708.
27. Pineles SL, Kraker RT, VanderVeen DK, Hutchinson AK, Galvin JA, Wilson LB, Lambert SR: Atropine for the Prevention of Myopia Progression in Children: A Report by the American Academy of Ophthalmology. Ophthalmology 2017, 124(12):1857-1866.
28. Qu J, Zhou X, Xie R, Zhang L, Hu D, Li H, Lu F: The presence of m1 to m5 receptors in human sclera: evidence of the sclera as a potential site of action for muscarinic receptor antagonists. Curr Eye Res 2006, 31(7-8):587-597.
29. Tan D, Tay SA, Loh KL, Chia A: Topical Atropine in the Control of Myopia. Asia Pac J Ophthalmol (Phila) 2016, 5(6):424-428.
30. Clark TY, Clark RA: Atropine 0.01% Eyedrops Significantly Reduce the Progression of Childhood Myopia. J Ocul Pharmacol Ther 2015, 31(9):541-545.
31. Gong Q, Janowski M, Luo M, Wei H, Chen B, Yang G, Liu L: Efficacy and Adverse Effects of Atropine in Childhood Myopia: A Meta-analysis. JAMA Ophthalmol 2017, 135(6):624-630.
32. Wu PC, Chuang MN, Choi J, Chen H, Wu G, Ohno-Matsui K, Jonas JB, Cheung CMG: Update in myopia and treatment strategy of atropine use in myopia control. Eye 2018.
33. Lee JJ, Fang PC, Yang IH, Chen CH, Lin PW, Lin SA, Kuo HK, Wu PC: Prevention of myopia progression with 0.05% atropine solution. J Ocul Pharmacol Ther 2006, 22(1):41-46.
34. Wu PC, Yang YH, Fang PC: The long-term results of using low-concentration atropine eye drops for controlling myopia progression in schoolchildren. J Ocul Pharmacol Ther 2011, 27(5):461-466.
35. Fang PC, Chung MY, Yu HJ, Wu PC. Prevention of myopia onset with 0.025% atropine in premyopic children. J Ocul Pharmacol Ther. 2010 Aug;26(4):341-5.
36. Mak CY, Yam JC, Chen LJ, Lee SM, AL. Y: Myopia: a review on regional epidemiology and methods of controlling myopia progression in children. Hong Kong Med J 2018, In Press.
37. Lam DS, Fan DS, Lam RF, Rao SK, Chong KS, Lau JT, Lai RY, Cheung EY: The effect of parental history of myopia on children's eye size and growth: results of a longitudinal study. Invest Ophthalmol Vis Sci 2008, 49(3):873-876.
38. Yam JC, Jiang YN: Childhood Myopia: Update on effective prevention. The Hong Kong Medical Diary 2016 Aug; 21(8):26-28) 2016, 21(8):26-28.
39. Lam CY, Tam PO, Fan DS, Fan BJ, Wang DY, Lee CW, Pang CP, Lam DS: A genome-wide scan maps a novel high myopia locus to 5p15. Invest Ophthalmol Vis Sci 2008, 49(9):3768-3778.
40. Ng TK, Lam CY, Lam DS, Chiang SW, Tam PO, Wang DY, Fan BJ, Yam GH, Fan DS, Pang CP: AC and AG dinucleotide repeats in the PAX6 P1 promoter are associated with high myopia. Mol Vis 2009, 15:2239-2248.
41. Tang SM, Lau T, Rong SS, Yazar S, Chen LJ, Mackey DA, Lucas RM, Pang CP, Yam JC: Vitamin D and its pathway genes in myopia: systematic review and meta-analysis. Br J Ophthalmol 2018.
42. Tang SM, Ma L, Lu SY, Wang YM, Kam KW, Tam POS, Young AL, Pang CP, Yam JCS, Chen LJ: Association of the PAX6 gene with extreme myopia rather than lower grade myopias. Br J Ophthalmol 2018, 102(4):570-574.
43. Khor CC, Miyake M, Chen LJ, Shi Y, Barathi VA, Qiao F, Nakata I, Yamashiro K, Zhou X, Tam PO, Cheng CY, Tai ES, Vithana EN, Aung T, Teo YY, Wong TY, Moriyama M, Ohno-Matsui K, Mochizuki M, Matsuda F, Nagahama Study G, Yong RY, Yap EP, Yang Z, Pang CP, Saw SM, Yoshimura N: Genome-wide association study identifies ZFHX1B as a susceptibility locus for severe myopia. Hum Mol Genet 2013, 22(25):5288-5294.
44. Shi Y, Gong B, Chen L, Zuo X, Liu X, Tam PO, Zhou X, Zhao P, Lu F, Qu J, Sun L, Zhao F, Chen H, Zhang Y, Zhang D, Lin Y, Lin H, Ma S, Cheng J, Yang J, Huang L, Zhang M, Zhang X, Pang CP, Yang Z: A genome-wide meta-analysis identifies two novel loci associated with high myopia in the Han Chinese population. Hum Mol Genet 2013, 22(11):2325-2333.
45. Cheung CY, Li J, Yuan N, Lau GYL, Chan AYF, Lam A, Tang FY, Tham CC, Pang CP, Chen LJ, Yam JC: Quantitative retinal microvasculature in children using swept-source optical coherence tomography: the Hong Kong Children Eye Study. Br J Ophthalmol 2018.
46. Ng DS, Cheung CY, Luk FO, Mohamed S, Brelen ME, Yam JC, Tsang CW, Lai TY: Advances of optical coherence tomography in myopia and pathologic myopia. Eye (Lond) 2016, 30(7):901-916.
47. Tan DT, Lam DS, Chua WH, Shu-Ping DF, Crockett RS, Asian Pirenzepine Study G: One-year multicenter, double-masked, placebo-controlled, parallel safety and efficacy study of 2% pirenzepine ophthalmic gel in children with myopia. Ophthalmology 2005, 112(1):84-91.

## Claims

1. A method for inhibiting or delaying the onset of myopia in a subject, comprising: administering to the subject a composition comprising about 0.025% atropine to about 0.05% atropine.

2. The method of claim 1, wherein the subject has a Spherical Equivalent (SE) of an eye of 0 to +1.0 D before administration of the composition.

3. The method of claim 2, wherein the SE is measured by cycloplegic autorefraction after administration of a cycloplegic agent.

4. The method of claim 1, wherein the composition comprises about 0.05% atropine.

5. The method of claim 1, wherein the composition is administered every other day, at least once daily, or at least twice daily.

6. The method of claim 1, wherein the atropine composition is administered to an eye of the subject.

7. The method of claim 6, wherein at least one drop, at least two drops, or at least three drops are administered to the eye.

8. The method of claim 7, wherein each drop contains about 20 µL to about 100 µL of the composition.

9. The method of claim 1, wherein the subject is at least about 4 years old.

10. The method of claim 9, wherein the subject is about 4 years old to about 9 years old.

11. The method of claim 1, wherein the administration continues for at least about six months, about one year, about two years, about three years, about four years, about five years, about six years, about seven years, or about eight years.

12. The method of claim 1, wherein the composition further comprises a pharmaceutically acceptable carrier or excipient.

13. The method of claim 12, wherein the pharmaceutically acceptable carrier or excipient comprises sodium phosphate monobasic, Hypromellose (HPMC), sodium chloride, boric acid, sodium borate, hydrochloric acid, or any combination thereof.

14. The method of claim 1, wherein the subject has no astigmatism or has astigmatism of less than 1.00 D before administration of the composition.

15. The method of claim 1, wherein the subject has no anisometropia or has anisometropia of less than 2.00 D before administration of the composition.

16. The method of claim 1, wherein the subject has at least one myopic parent.

17. The method of claim 16, wherein the myopic parent has an SER of less than or equal to -3.00 D.

18. The method of claim 1, wherein the onset of myopia is delayed for at least about 1 month, about 2 months, about 3 months, about 4 months, about 5 months, about 6 months, about 12 months, about 18 months, about two years, about three years, about 4 years, about five years, about six years, about 7 years, or about eight years.
